Europäisches Patentamt

**European Patent Office**

Office européen des brevets

(11) Publication number: **0 312 164**
**A1**

## (12) EUROPEAN PATENT APPLICATION

(21) Application number: **88202242.9**

(22) Date of filing: **07.10.88**

(51) Int. Cl.⁴: **C12P 21/00 , C12N 15/00 , A61K 39/245**

(30) Priority: **16.10.87 US 109986**
**16.10.87 US 109983**
**16.10.87 US 109984**

(43) Date of publication of application:
**19.04.89 Bulletin 89/16**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Lehman, E. Dale**
**104 Crestwood Drive**
**Lansdale, PA 19446(US)**
Inventor: **Sha, Amanda T.**
**18 Cool Valley Road**
**Malvern, PA 19355(US)**
Inventor: **Friedman, Arthur**
**121 Frog Hollow Road**
**Churchville, PA 18966(US)**
Inventor: **Mudri, Mary**
**1735 Meadow Glen Drive**
**Lansdale, PA 19446(US)**

(74) Representative: **Hesketh, Alan, Dr.**
**European Patent Department Merck & Co.,**
**Inc. Terlings Park Eastwick Road**
**Harlow Essex, CM20 2QR(GB)**

(54) **Purification of recombinant epstein-barr virus antigens from vero cells, yeast cells or L cells.**

(57) Recombinant Epstein-Barr virus membrane antigens are purified by steps adapted to a Vero cell expression system, including lectin-affinity chromatography on a lentil lectin column, and gel filtration chromatography.

Recombinant Epstein-Barr virus membrane antigens are also purified by steps adapted to a yeast expression system, including lectin-affinity chromatography on a lentil lectin column followed by anion exchange chromatography.

Recombinant Epstein-Barr virus membrane antigens are also purified by steps adapted to a mouse L cell expression system, including lectin-affinity chromatography on a lentil lectin column, followed by anion exchange and gel filtration chromatography.

# PURIFICATION OF RECOMBINANT EPSTEIN-BARR VIRUS ANTIGENS FROM VERO CELLS, YEAST CELLS OR L CELLS

## BACKGROUND OF THE INVENTION

The etiological agent of infectious mononucleosis is the Epstein-Barr virus (EBV), a member of the herpes virus group. The disease occurs in persons with no prior EBV antibody titer. EBV-specific antibodies can be demonstrated early after onset. Antibody titers decline during convalescence, but remain detectable for life, correlating with immunity to the disease. The virus is regularly present in the oropharyngeal secretions of patients with infectious mononucleosis and often persists for months after acute disease. As with other herpesgroup viruses, a persistent carrier state follows primary EBV infection.

The disease is spread through close contact, mainly by oral secretions. In areas of poor sanitation and hygiene, primary EBV infections usually occur in infancy and are silent or too mild to be diagnosed. In higher socioeconomic groups, primary exposure to EBV is often delayed until adolescence or later, when infections usually lead to typical infectious mononucleosis.

The fact that EBV transforms lymphocytes into rapidly dividing cells indicates that it may be oncogenic. There is strong evidence that EBV is involved in the etiology of Burkitt's lymphoma and nasopharyngeal carcinoma.

Prevention of EBV infection by vaccination can be achieved by immunization with EBV antigens displayed on the outer surface of the viral lipid envelope and of virus-infected cells. Glycoproteins of 350, 220 and 85 KDa mobilities (gp350, gp220, gp85) have been identified in these locations. Immunizations of primates with gp350 and gp220 prevent infection on challenge with virus. There is also published evidence that gp350 and gp220 are involved with the specific adsorption of EBV to the surface of immunoglobulin producing lymphocytes. Finally, antibodies of monoclonal or polyclonal origin with specificites to gp350 or gp220 are known to neutralize virus infectivity. All of this evidence specifically implicates the immunological recognition of gp350 or of gp220 in the control and prevention of EBV infections.

The two major Epstein-Barr membrane antigens, gp350 and gp220, are encoded by the same EBV DNA open reading frame. While gp350 is encoded by the continuous open reading frame, gp220 is encoded by two exons of the same frame therein. This close relationship between gp350 and gp220 is consistent with immunological evidence that monoclonal antibodies frequently react with both gp350 and gp220.

From their nucleotide sequence, some important features of these proteins can be deduced, i.e., the primary translation product of gp350 is 907 amino acids, while that of gp220 is 710 amino acids. In addition, an 850 amino acid region in gp350 and a 653 amino acid region in gp220 are between putative signal and anchor sequences. These central regions are serine- and threonine-rich and contain 36 (gp350) or 25 (gp220) asparagine-x-serine or -x-threonine sequences, which may be signals for N-linked glycosylation. Those regions having potential glycosylation sites are likely to be on the outer surface of the membrane.

Both gp350 and gp220 are known to be extensively glycosylated, since each glycoprotein has substantially anomalous electrophoretic mobility. The protein core still accounts for less than half of the apparent 350 and 220KDa sizes of the fully glycosylated glycoproteins. Gp350 is extensively modified with both N-linked and 0-linked glycosyl side chains since treatment with N-glycanase to remove complex and high mannose N-linked sugars results in a apparent size of 230 KDa, while the apparent size of the completely unglycosylated gp350 precursor is 135 KDa.

The unusually extensive glycosylation of gp350 and gp220 clearly influences their structure, immunogenicity, and their role in disease resistance. When gp350 DNA sequence was expressed in and purified from E. coli, an antibody response could be induced in rabbits which neutralized EBV. However, multiple injections of substantial quantities of protein were required to elicit this response, an indication of poor immunogenicity due to the absence of glycosylation.

Present applicants have shown that expression of gp350 DNA sequences in recombinant eukaryotic cells results in glycosylated products (rEBMA) which, when injected into rabbits three times in Freund's adjuvant, induces antibodies that react with the homologous antigen and with the native, non-recombinant antigen obtained from B95-8 lymphoid cells. All rabbits also produced antibodies that reacted with the surface of EBV positive lymphoid cells, as determined by indirect immunofluoresence. Additionally, all the mammalian cell-derived expression products induced antibodies that neutralize Epstein-Barr virus in vitro.

The present invention is drawn to methods of purifying gp350 or gp220 expressed by recombinant eukaryotic cells for the purpose of preparing suitable vaccines. It is both uncommercial and impractical to make vaccines from infected lymphocytes, e.g. B95-8 cells. Recombinant eukaryotic cell systems provide the most appropriate vehicle for expressing gp350 or gp220 with structural and immunological characteristics most closely resembling the native or natural form in disease states caused by EBV. A vaccine containing an extensively glycosylated antigen such as gp350 or gp220 is more effective if the purification methods of the present invention are performed. The present invention is a method of purifying rEBMA without immunoaffinity chromatography, and without denaturing conditions with e.g. urea or SDS.

Mammalian or yeast cells expressing gp350 or gp220 are disrupted, and the cell disruptate is subjected to one or more steps of lectin affinity chromatography followed by anion-exchange or gel filtration chromatography. Further steps such as gel filtration may be required, as the case may be. Each method has been specifically adapted to the cell type employed.

The present invention is amenable to scale-up for producing large quantities of antigen suitable for commercially produced vaccines. Once the antigen is purified, it can be used for preparing vaccines, for conducting immunochemical tests of a wide variety, and for making diagnostic tests for antibodies to EBV. The utility, value and feasibility of the present invention is specifically illustrated by the purification of rEBMA from transfected Vero cells, a cell line previously employed for the production of polio vaccine. It is also specifically illustrated for transformed yeast cells and transfected mouse L cells.

Purification of glycosylated recombinant proteins not infrequently requires new methods or novel combinations of old methods, since the composition of the source for recombinant forms, e.g., transfected mammalian cells, is different from conventional sources. Not only is the protein foreign to the cell but also the protein is glycosylated in a different manner. One cannot predict what methods are useful for isolating proteins from recombinant cell cultures or recombinant mammalian or yeast expression systems on the basis of the knowledge and know-how of methods useful for isolating proteins from classical or otherwise conventional sources. In addition, variations in glycosylation of proteins are correlated with species, tissue, or cell line specific differences. [Kornfeld, R. et al. Ann. Rev. Biochem. 54, 631 (1985)]. Purification processes designed for preparing vaccines require unusual purity in the product, another indication of the unpredictability in the art. For a similar view, see U.S. Patent 4,624,918.

A process is disclosed for purifying recombinant EBV membrane antigens from a recombinant mammalian expression system, comprising the steps of:

(a) disrupting VERO cells expressing recombinant EBV membrane antigens;

(b) subjecting the disruptate to one or two steps of lectin affinity chromatography, yielding eluted fractions containing EBV membrane antigen;

(c) subjecting said eluted fractions to gel filtration chromatography, yielding purified recombinant EBV membrane antigens. Additional steps in the process may be required. This process is designed for scale-up in the commercial production of vaccines.

A second process is disclosed and is suitable for purifying recombinant EBV membrane antigens secreted from a recombinant yeast expression system, comprising the steps of:

(a) collecting and dialyzing a culture medium contacted with yeast cells secreting rEBMA; yielding a retentate containing rEBMA;

(b) subjecting said retentate to one or two steps of lectin affinity chromatography, yielding eluted fractions containing rEBMA;

(c) subjecting said eluted fractions to anion exchange chromatography, yielding purified yeast-derived rEBMA. Additional steps in the process may be required. This second process is designed for scale-up in the commercial production of vaccines.

A third process is disclosed and is suitable for purifying recombinant EBV membrane antigens from a recombinant mammalian expression system comprising the steps of:

(a) disrupting mouse L cells expressing recombinant EBV membrane antigens;

(b) subjecting the disruptate to one or two steps of lectin affinity chromatography, yielding eluted fractions containing EBV membrane antigen;

(c) subjecting said eluted fractions to anion exchange chromatography, yielding chromatographed fractions containing recombinant EBV membrane antigens; and

(d) subjecting said chromatographed fractions to gel filtration chromatography, yielding purified recombinant EBV membrane antigens (rEBMA). Additional steps in the process may be required. This third process is designed for scale-up in the commercial production of vaccines.

## BRIEF DESCRIPTION OF THE INVENTION

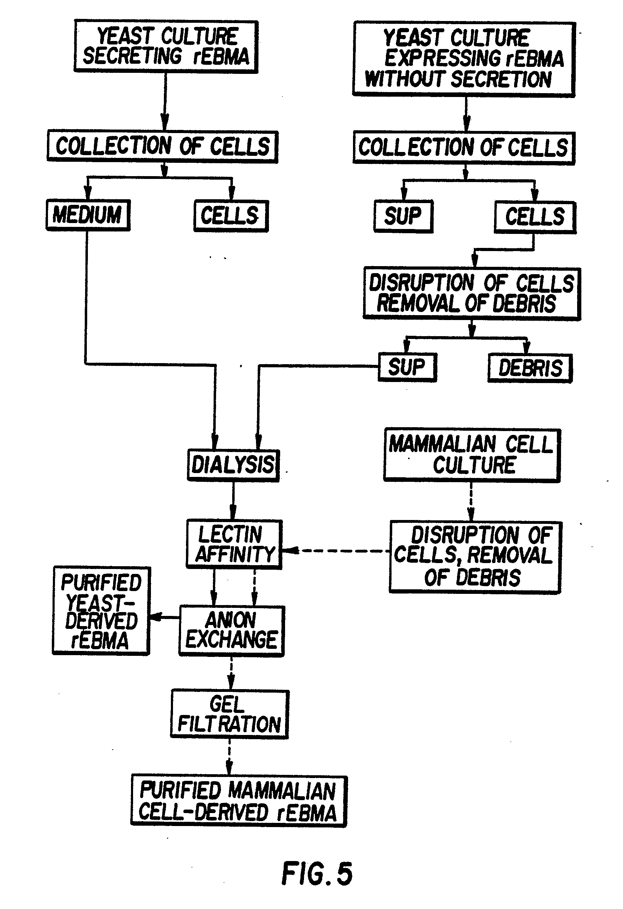

Figure 5 Scheme for purifying rEBMA.

## BRIEF DESCRIPTION OF THE DRAWINGS

Figure 1A Map of plasmid pMA102. An EBV DNA fragment beginning 10bp upstream of the gp350 open reading frame (represented by the open box) and ending 2Kbp downstream of the gp350 mRNA polyadenylation site was inserted into the polylinker of pUC19 to yield pMA102.

Figure 1B Map of plasmid pMAΔ-gpt. pMA delta-gpt is pSV2gpt containing the EBV gp350 DNA fragment of pMA102. An oligonucleotide containing a Bc1I site and stop codons in all three frames has been inserted into a Sca1 site located at the beginning of the coding region for the hydrophobic transmembrane domain.

Figure 2 Assembly of VZV gpl promoter/EBV gp350 gene constructs for Vero cells.

A. The locations of the VZV gpl promoter and the EBV gp350 gene in their respective viral genomes are shown, as well as enlargements.

B. Map of VZV P, L (gpl)-gp350. The arrangement in the fusion gp350 construct is shown. The VZV gpl promoter (P) and leader sequence (L) of VSV gpl protein up to codon 35 are joined in frame to the gp350 sequence at codon 21.

C. Map of VZV P(gpl)-gp350. The arrangement in the non-fusion gp350 construct is shown. The VSV gpl promoter sequence extends from the ECORV site up to within 2 bp of the original VZV gpl ATG. Additional bases are inserted to link up sequences for the gpl promoter and gp350.

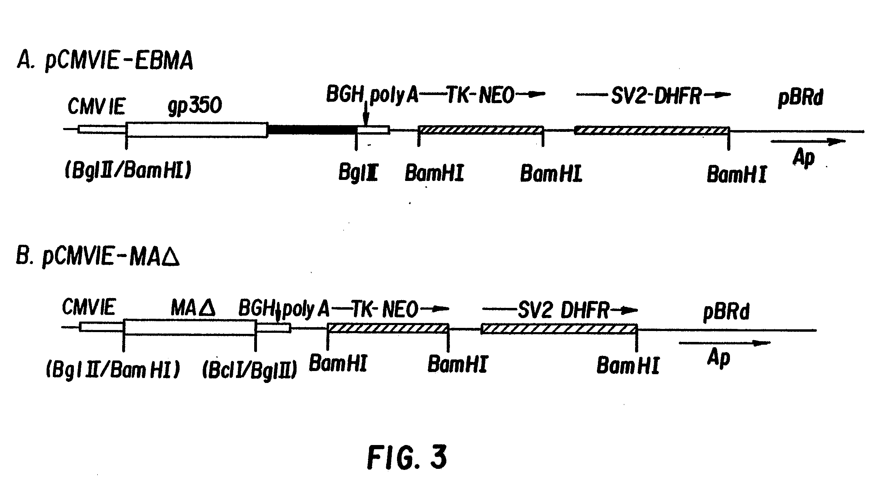

Figure 3. Map of cytomegalovirus/gp350 constructs for mouse Ltk⁻aprt⁻cells.

A. pCMVIE-EBMA is the EBV gp350 DNA fragment of pMA102 inserted into a Bg1II site downstream of the cytomegalovirus immediate early transcriptional promoter in pCMVIE-AK1-DHFR. This expression vector contains two selectable marker genes, the neomycin (G418) resistance gene and the dihydrofolate reductase (DHFR) gene.

B. pCMVIE-MA delta contains the EBV gp350 DNA sequence derived from pMA delta-gpt and inserted into pCMVIE-AK1-DHFR.

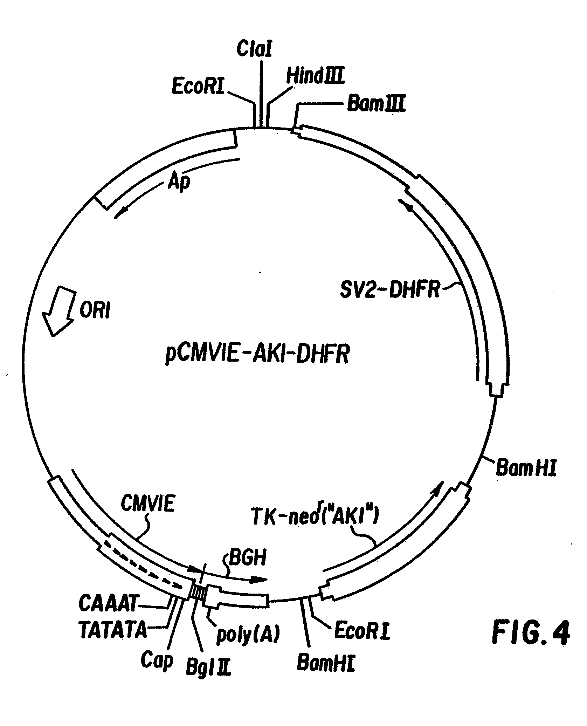

Figure 4 pCMVIE-AK1-DHFR is the mammalian expression vector into which EBV gp350 DNA sequences are inserted to obtain pCMVIE-EBMA or pCMVIE-MA delta.

## ABBREVIATIONS AND DEFINITIONS

CHAPS: 3-[(3-cholamidopropyl)-dimethylammonio]-1-propanesulfonate.

Δ: delta

EA: early antigen

EBMA: Epstein-Barr virus membrane antigen from any source, including EBV antigens displayed on the outer surface of viral envelopes or of EBV-infected cells.

EBNA: EBV nuclear antigen

EBV: Epstein-Barr virus

IR1: Internal Repeat 1

IRS: Internal Repeat Sequence

gp350: EBMA or rEBMA with apparent electrophoretic mobility of 350 Kilodaltons, also known as gp300/350.

gp220: EBMA or rEBMA with apparent electrophoretic mobility of 220 Kilodaltons, also known as gp200/250.

gp: glycoprotein

KDa: Kilodaltons

Kbp: Kilobase pairs

L: Leader

P: promoter

Recombinant EBV: EBV coded by a vector, or by sequences derived therefrom, wherein the vector is constructed by recombinant DNA technology.

Recombinant eukaryotic expression system: A eukaryotic cell containing a foreign DNA expressing a foreign protein or foreign oligopeptide.

rEBMA: recombinant Epstein-Barr virus membrane antigen or antigens (or portion thereof) coded by foreign DNA and expressed in a recombinant eukaryotic expression system.

recombinant protein: A polypeptide or oligopeptide expressed by foreign DNA in a recombinant eukaryotic expression system, e.g. rEBMA in Vero cells or yeast cells.

SDS - PAGE: polyacrylamide gel electrophoresis in gels containing sodium dodecyl sulfate

TRS: Terminal Repeat Sequence

UL: Unique long sequence

US: Unique short sequence

VCA: viral capsid antigen

VZV: varicella-zoster virus

## DETAILED DESCRIPTION OF THE INVENTION

Methods have been designed to purify recombinant EBMA gp350 or gp220 antigen from recombinant eukaryotic expression systems. The details of each method vary with the cell line or expression system as source of the antigen. In general, the gp350 or gp220 antigen isolate was prepared from extracts by lectin affinity chromatography. After elution with the appropriate ligand, e.g. α-methyl mannoside, anion exchange or gel filtration chromatography, or both, is (are) conducted. Material eluted at appropriate salt concentrations was about 90% or more in purity. The specific protocol is adapted to the particular expression system employed.

It will be understood that the novel preparation processes of the present invention are applicable to a range of expressed recombinant EBMA, specifically gp350 and gp220. The processes of the present invention are also designed to provide rapid and efficient methods of preparing vaccines containing variants of gp350 or gp220. For example, conservative substitutions in the amino acid sequence [defined as sets in Table 1 of Taylor, W.R., J. Mol. Biol. 188, 233 (1986)] generally will not result in any substantial or novel modification of the principles and practice of the present invention. Alternatively, deletions within the coding regions will not in general require any modifications of the processes for preparing vaccines discussed herein. It will be understood that rEBMA, gp350 or gp220 in this application each includes any such variations or portions of the amino acid sequence, whether by conservative amino acid substitution, deletion, or other process, provided that the resulting rEBMA or portion thereof, is immunochemically reactive with antibodies specific for gp350, gp220, Epstein-Barr virus, or other natural and native forms of the Epstein-Barr membrane antigen amino acid sequences.


Eukaryotic Expression Systems


It is now a relatively straightforward technology to prepare eukaryotic cells expressing a foreign gene. Such eukaryotic cells act as hosts and include yeasts, fungi, plant cells and animal cells. Expression vectors for many of these host cells have been isolated and characterized, and are used as starting materials in the construction, through conventional recombinant DNA techniques, of vectors having a foreign DNA insert of interest. Any DNA is foreign if it does not naturally derive from the species of host cells used to express the DNA insert. The foreign DNA insert may be expressed on extra-chromosomal plasmids or after integration in whole or in part in the host cell chromosome(s), or may actually exist in the host cell as a combination of more than one molecular form. The choice of host cell and expression vector for the expression of a desired foreign DNA largely depends on availability of the host cell and how fastidious it is, whether the host cell will support the replication of the expression vector, and other factors readily appreciated by those of ordinary skill in the art, such as the splicing of secretory sequences onto the structural gene insert for the purposes of gaining simpler modes of purification of the desired recombinant protein.

Yeast expression systems, which are one variety of recombinant eukaryotic expression systems, generally employ Saccharomyces cerevisiae as the species of choice for expressing recombinant proteins. However, it will be apparent to those skilled in the art that, for the expression of rEBMA, the selection of a host strain or cell extends to species from other genera of yeast from the Families Saccharomycetaceae and Cryptococcaceae, including, but not limited to Pichia, Candida, Hansenula, Torulopsis, Kluyveromyces, and Saccharomycopsis. Similarly, selection of a host strain or cell extends to other species of the genus Saccharomyces. Many of the species of this genus are capable of interbreeding with S. cerevisiae and are likely to possess promoters which are analogous or identical to promoters in S. cerevisiae, including but not limited to GAL10, ADH2, and/or alpha mating factor promoters. Therefore, it will be apparent to those skilled in the art that, for the expression of recombinant EBV polypeptides, the selection of a host strain extends to other species of the genus Saccharomyces, including but not limited to carlsbergensis, uvarum, rouxii, montanus, kluyveri, elongisporus, norbensis, oviformis, and diastaticus.

Yeast vectors useful for constructing recombinant yeast expression systems for expressing rEBMA include, but are not limited to, shuttle vectors, cosmid plasmids, chimeric plasmids, and those having sequences derived from 2-micron circle plasmids. A variety of promoters and other yeast transcriptional control sequences may be used to express the inserted DNA sequence coding for EBMA, including those of GAL10, ADH I or ADH II, and the alpha mating factor. The following list of yeast vectors is intended for illustrative purposes only:
pAAH5
pAH5
pAAR6
pFRPn
pGAL 10-MFα1
pJC197
pMA56
pABADE8
pAXα11
pAH9
pAH10
pAH21
pMAC561

pLG669
pMA91
pMA301
pYEHBs
pAM82.

Insertion of the appropriate DNA sequence coding for rEBMA, or portion thereof, into these vectors can, in principle, result in a useful recombinant yeast expression system for rEBMA where the modified vector is inserted into the appropriate host cell, by transformation or other means. Since yeast recombinant EBMA is glycosylated, lectin affinity chromatography is an appropriate method for commercial purification of the expressed protein.

Recombinant mammalian expression systems are another means of producing the recombinant Epstein-Barr Virus membrane antigens of the present invention. In general, a host mammalian cell can be any cell that has been efficiently cloned in cell culture. An expression vector for mammalian cells should have a selectable marker, such as resistance to certain antibiotics in the neo gene, so that the presence of the vector in the cell can be detected. DNA-mediated transfection, transvection or transformation of the cell can be accomplished by, e.g., the calcium phosphate technique or microinjection.

Host mammalian cells useful for the purposes of constructing a recombinant mammalian expression system include, but are not limited to, Vero cells, NIH3T3, GH3, COS, murine C127 or mouse L cells. Mammalian expression vectors can be based on virus vectors, plasmid vectors which may have SV40, BPV or other viral replicons, or vectors without a replicon for animal cells. Detailed discussions on mammalian expression vectors can be found in Elder, J.T. et al. Ann. Rev. Gen 15, 295 (1981); Gluzman, Y. (ed.) "Eukaryotic Viral Vectors" Cold Spring Harbor Laboratory 1982; Pouwels, P.H. et al. "Cloning Vectors: A Laboratory Manual" Elsevier 1985; Rigby, P.W.J. J. Gen. Virol. 64, 255 (1983); and Subramani, S. et al. Anal. Biochem. 135, 1 (1983).

## PURIFICATION OF rEBMA

To combat instability and breakdown of rEBMA during purification procedures, buffers generally incorporate protease inhibitors such as phenylmethylsulfonylfluoride (PMSF) or EDTA. The preferred inhibitor is PMSF at a final concentration of about 2mM.

Typical protease inhibitors suitable for the procedures and protocols of this invention include but are not limited to metal-chelating agents, heavy metal ions, SH blocking reagents, substrate-like compounds of proteases or polypeptides that inhibit proteases. Some useful protease inhibitors are provided as follows:

$Ag^{++}$, $Hg^{++}$, $Cu^{++}$ and other heavy metal ions
antithrombin III
antithrombin III - heparin
$\alpha_1$-antitrypsin
aprotinin
basic amino acids
benzamidine
bestatin
$\alpha,\alpha'$-bipyridyl, Na-fluoride
4-bromo-phenacyl bromide
chicken egg white trypsin inhibitor
chymostatin
citrate
cysteine
4-dinitrophenol diethylphosphate
DFP (diisopropylphosphofluoridate)
DTT (dithiothreitol)
E-64 (Boehringer Mannheim)
EDTA and other chelating agents
formaldehyde
guanidinium chloride
heparin
hirudin
4-hydroxymercuribenzoate
iodoacetamide
iodoacetic acid
leupeptin
$\alpha_2$-macroglobulin
mercaptoethanol
p-mercuribenzoate
mercuric chloride
$\alpha$-microglobulin
$\alpha$-N-(p-nitrobenzyl-oxycarbonyl)-L-arginylchloromethyl ketone
oxalate
pepstatin from a variety of sources, including

Streptomyces

1,10-phenanthroline
2-phenanthroline
phenothiazine-N-carbonyl chloride
phosphoramidone
PMSF
pyrophosphate
SH-blocking reagents
silver nitrate
soybean trypsin inhibitor
p-toluene sulfonyl fluoride
TLCK(L-1-chloro-3-(4-tosylamido)-7-amino-2-heptanonehydrochloride)
Triton X-100 and other detergents at low concentrations
TPCK(L-1-chloro-3-(4-tosylamido)-4-phenyl-2-
butanone)
trypsin inhibitor from hen egg
ZPCK (benzyloxycarbonyl-L-phenylalanine)

Buffered solutions containing one or more of the listed inhibitors may be adapted to one or more steps in the process of purifying rEBMA.

INITIAL PURIFICATION BEFORE LECTIN

AFFINITY CHROMATOGRAPHY

A. Non-secreted rEBMA in yeast can be isolated as follows. See Figure 5.

Yeast cells transformed with expression vectors coding for and expressing gp350/220 protein, or variants thereof, are grown and harvested. If storage of the cells is desired, the cells are then washed in buffer, e.g. PBS, to form a cell paste. Storage of the cell paste is typically done by freezing in liquid $N_2$.

Purification typically begins as follows. A batch of frozen cell paste is thawed and suspended in buffer containing proteolytic inhibitors, e.g. PBS in 2mM PMSF, or hypertonic phosphate buffers. Cells are then disrupted, preferably by mechanical means.

Yeast cell disruption results in a crude extract. It is necessary at this point to remove cell debris in the extract so that mechanical clogging in subsequent purification steps is avoided. Centrifugation is the preferred method of debris removal, e.g. at 4,400 xg for 5 minutes or a variety of other speeds and lengths of time.

Before applying the supernatant to a lectin affinity column, it may be desirable to concentrate, then diafilter or dialyze . Alternatively or additionally, anion exchange chromatography or density gradient centrifugation may be performed.

B. Initial purification of secreted rEBMA in yeast may be performed by the following procedures. See Figure 5.

Yeast cells transformed with expression vectors coding and expressing gp350 or gp220 protein, or variants thereof, are grown and harvested. Secretory proteins released into the culture medium are treated by first removing the yeast cells by e.g, centrifugation. Additional or alternative steps of concentration, diafiltration or dialysis may be performed before application to a lectin affinity column.

C. For mammalian cells expressing rEBMA, (Figure 5), cells are disrupted with any one of a variety of procedures, including but not limited to freezing and thawing, exposing to high pH, treating with a chaotropic agent, or extracting with a detergent. The preferred disruption technique is to use a detergent, most preferably octylphenoxy polyethoxyethanol (marketed under the trademark TRITON X-100) at a final concentration of about 0.5 to about 2% (w/v).

After disruption of the mammalian cells, cell debris is generally removed by centrifugation and the clarified supernatant may be ready for application of lectin affinity chromatography. Alternatively, additional steps of concentration, diafiltration or dialysis may be performed on the clarified supernatant before passing the sample through a lectin affinity column.

LECTIN AFFINITY CHROMATOGRAPHY

Affinity chromatography with insolubilized lectin can be carried out with a variety of lectins, including lectins derived from peanut, castor bean, wheat germ, jack bean, lentil, and the garden pea. In principle, any lectin with a specificity for the sugar residues of glycosylated rEBMA can be employed, such as lectins specific for N-acetyl glucosamine, mannose, galactose or sialic acid. The preferred lectins bind $\alpha$-D-mannose, and include concanavalin A and the lentil lectin, the most preferred being lentil lectin. Alternatively, these and other lectins can be employed for the purpose of binding undesired glycoproteins for removal. The techniques of affinity chromatography with insolubilized lectin can be performed according to Kristiansen, T., Meth. Enzymology 34, 331 (1974). The substitution of lentil lectin with other solid-phase lectin adsorbents will be readily apparent, in view of the wide variety of available lectins for chromatographic purposes. See, for example, Sharon, N. et al. Science 177, 949 (1972) Lis, H. et al. Ann. Rev. Biochem. 42, 541 (1973) and Lis, H. et al. Ann. Rev. Biochem 55, 35 (1986).

ANION EXCHANGE CHROMATOGRAPHY

In most cases, it is desirable to conduct at least one step of anion exchange chromatography after lectin affinity chromatography. Typically, fractions of glycoprotein bound to the solid-phase lectin are eluted, either by ligand competition or by denaturants, and subjected to ion exchange chromatography on a resin, gel or matrix with positive charge. Typical anion exchange matrices include, but are not limited to,
DEAE cellulose
DEAE agarose
DEAE Biogel
DEAE dextran
DEAE Sephadex
Aminohexyl Sepharose
Ecteola cellulose
TEAE cellulose
QAE cellulose
MONO-Q, or
Benzoylated diethylaminoethyl cellulose.

The preferred anion exchanger is marketed under the trademark MONO-Q (Pharmacia). General background information on ion exchange chromatography can be found, for example, in E. A. Peterson, "Cellulosic Ion Exchangers" in Work, T.S. et al. Laboratory Techniques in Biochemistry and Molecular Biology Volume 2, Part II, pages 223 et seq. North-Holland 1970.

Anion exchange chromatography can be added as an additional step to any point of the purification process of rEBMA. It has been found to be unnecessary in the purification of rEBMA expressed in Vero cells.


## GEL FILTRATION CHROMATOGRAPHY


After lectin affinity chromatography, or, alternatively, after anion exchange chromatography, a step of gel filtration is usually necessary before a product rEBMA of satisfactory purity has been isolated. Numerous types of gel filtration matrices can be employed. See, for example, Fischer, L., "Gel Filtration Chromatography," in Work, T. S. et al. Laboratory Techniques in Biochemistry and Molecular Biology Elsevier 1980. In the case of some recombinant yeast expression systems, gel filtration has been found to be unnecessary.

While the particular sequence of chromatographic steps of, firstly, lectin affinity, followed secondly by one or both of anion exchange and gel filtration, are the typical protocols for purifying rEBMA in this invention, it will be understood that the sequence can be varied. Futhermore, anion exchange chromatography can precede lectin affinity chromatography.


## ADDITIONAL STEPS


Other conventional or known steps normally used in purification of recombinant proteins may be added to the process of purifying rEBMA. These steps include, but are not limited to:

(a) selective adsorption or partition on a solid-phase, e.g. silica gel, calcium phosphate, charcoal, or celite alumina;

(b) hydrophobic interaction chromatography with, e.g. butyl agarose;

(c) selective extraction with solvents or reagents. Extraction with other solvents or reagents for other purposes e.g., solubilization, may be needed in different steps. See, for example, Hjelmeland, L.M. et al. Meth. Enzym. 104, 305 (1984).

(d) Precipitation with salts is also included, e.g., ammonium sulfate, or on a pH gradient by isoelectric precipitation;

(e) chromatography by any standard method, including paper, thin-layer, gel, molecular sieve, molecular exclusion, cation-exchange, ligand affinity, immunoaffinity, or by electrophoresis;

(f) solvent fractionation by two phase extractions, e.g. with PEG and dextran [Anderson, E. et al., Ann. N.Y. Acad. Sci. 413, 115 (1983)].

(g) dialysis, ultrafiltration, or diafiltration;

(h) density-gradient centrifugation;

(i) electrofocusing;

(j) freeze drying, lyophilization; or

(k) crystallization.

This list is by no means exhaustive. Its order is not an indication of the preferred order of purification. It will be understood that a successful purification of rEBMA may be conducted with any or all of steps (a)-(k), in conjunction with lectin affinity chromatography and gel filtration chromatography. Steps (a)-(k), as well as other purification techniques, may be inserted as an additional step or steps at any point or points of the purification process.


## EXAMPLES


### A. Materials and Methods


Identification of EBMA, rEBMA, or other proteins, and assessment of purity in all cases was done by polyacrylamide gel electrophoresis in 0.1% sodium dodecyl sulfate (SDS-PAGE) followed by silver staining and protein immunoblotting. The samples to be assayed were concentrated about ten-fold with microconcentrators. Duplicate aliquots were mixed with an equal volume of a buffer containing 4% sodium dodecyl sulfate (SDS), 0.25 M Tris-HCl, pH 6.8, and 0.20 M dithiothreitol and incubated 15 minutes at 100°C. For immunoblot analysis [Burnette, W.N. Anal. Biochem. 112, 192 (1981)], samples were electrophoresed through 7.5% polyacrylamide separating gels [Laemmli U, et al. Nature 227, 680 (1970)], transferred to nitrocellulose and incubated with high-titer EBV-immune human serum or with a mouse monoclonal antibody against the gp350 polypeptide expressed in E. coli, and rabbit anti-mouse IgG antibody (Cappel). Blots were developed with $^{125}$I-protein A (Amersham). All incubations and washes were carried out in a solution designated BLOTTO [Johnson, D. et al. Gene Anal. Techn. 1, 3 (1984); 10% w/v Nonfat Dry Milk, 0.9% w/v NaCl, 10mM Tris pH 7.5]. Silver staining of polyacrylamide gels was done by the method of Morrissey [Anal. Biochem. 117, 307-310 (1981)].

Alternatively, proteins were measured by the protein dot blot assay, as follows. To assay secreted gp350, clarified culture medium was mixed with 1/10 volume on 1% 3-[(3-cholamidopropyl)-dimethylammonio]-1-propane sulfonate [CHAPS, SerVa] in TBS (10mM Tris pH 7.6, 250mM NaCl). An equal volume of 2 x TBS with 40% methanol was then added. For assay of intracellular gp350, cells were resuspended in an equal volume of 2 x CHAPS lysis buffer (200mM sodiumHEPES buffer, pH 7.5, 300mM NaCl, 20mM EDTA, 1% CHAPS). After 10 minutes at 25°C and a 5 minute centrifugation in a microfuge, supernatants were diluted with 4 volumes of 1 x TBS/20% methanol. Samples (100ul) were then applied to nitrocellulose which had been pre-equilibrated in 1 x TBS/20% methanol in a 96-well dot blot apparatus. Each well was washed with 500ul of 1 x TBS/20% methanol. The nitrocellulose was air-dried for one hour and then incubated for 2 hours at 37°C in BLOTTO. High-titer EBV-immune human serum (VCA titer 1:10,000, EA titer 1:10,000 and EBNA titer 1:160) was added to the BLOTTO and incubated at room temperature for 18 hours. The filter was then washed twice in blot wash solution (50mM Tris pH 7.5, 250mM NaCl, 3mM EDTA, 0.5% polyoxyethylene (20) sorbitan monolaurate) for one hour at 37°C and incubated for 2 hours in affinitypurified $^{125}$I-protein A (Amersham, 2 x $10^5$cpm/ml) in BLOTTO at 37°C. After two more 1 hour washes at 37°C, filters were dried for 1 hour and exposed to x-ray film. Known amounts of affinitypurified gp350 were used as standards in dot blot assays. Because of its extensive glycosylation, gp350 protein determinations are only approximate. Furthermore, the efficiency of detecting gp350 and gp220 varied among immunoassays with seemingly identical techniques.

For analysis of fixed cells by indirect immunofluoresence, transfected cells were grown on a chamber slide (Miles), fixed in acetone or methanol at -20°C for 10 minutes, incubated with ascites fluid of a mouse monoclonal antibody specific for gp350 [1:200 dilution in phosphate-buffered saline (PBS) and 5% goat serum], biotinylated goat anti-mouse IgG (1:200 dilution, BRL) and fluorescein-conjugated streptavidin (1:200 dilution, BRL). Slides were observed and photographed with a photomicroscope equipped with epifluorescence.

## B. Plasmid constructions

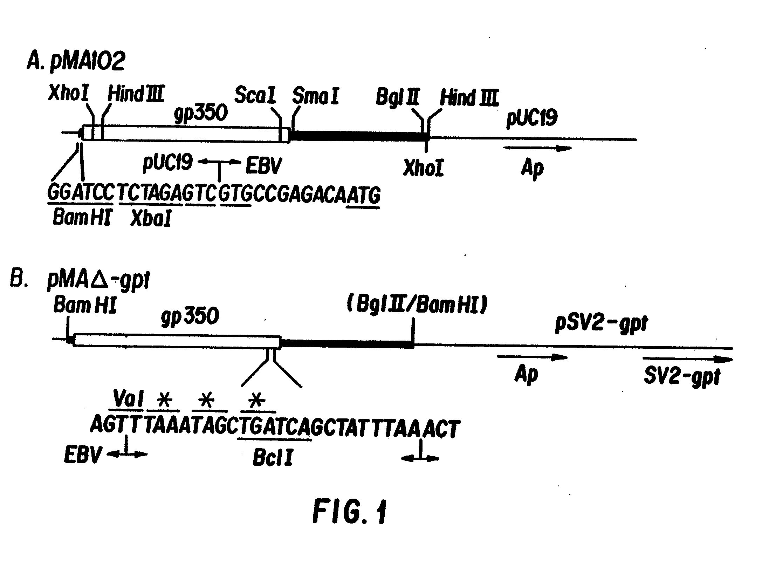

The EBV gp350 coding domain was subcloned from the EBV DNA BamHI L fragment [Dambaugh, T. et al. Proc. Natl. Acad. Sci. 77, 2999 (1980)] into pUC19 [Norrander, J. et al. Gene 26, 101 (1983)] in two steps. First, the 340 bp BanI-HindIII fragment coding for the N-terminus was inserted between the pUC19 HincII and HindIII sites. Then a 4.2 Kbp XhoI fragment, which begins at the XhoI site within the 340 bp BanI-HindIII fragment and contains the rest of the gp350 coding region, was inserted into the cloned BanI-HindIII fragment to recreate the complete coding sequence in pUC19 (pMA102, Fig 1A). To construct the gp350 gene with a stop codon inserted before the carboxy-terminal transmembrane anchor sequence (pMA-delta-gpt Fig. 1B), the 4.4 Kbp BamHI-Bg1II fragment of pMA102 containing the complete gp350 open reading frame was first inserted into the BamHI site of pSV2-gpt [Mulligan, R.C. et. al. Proc. Natl Acad. Sci 78, 2072 (1981)] to yield pMA-gpt. A synthetic oligonucleotide containing stop codons in three reading frames and a Bc1I site was then inserted into the ScaI site of the gp350 gene which interrupts the gene 8 bp after the beginning of the membrane anchor domain.

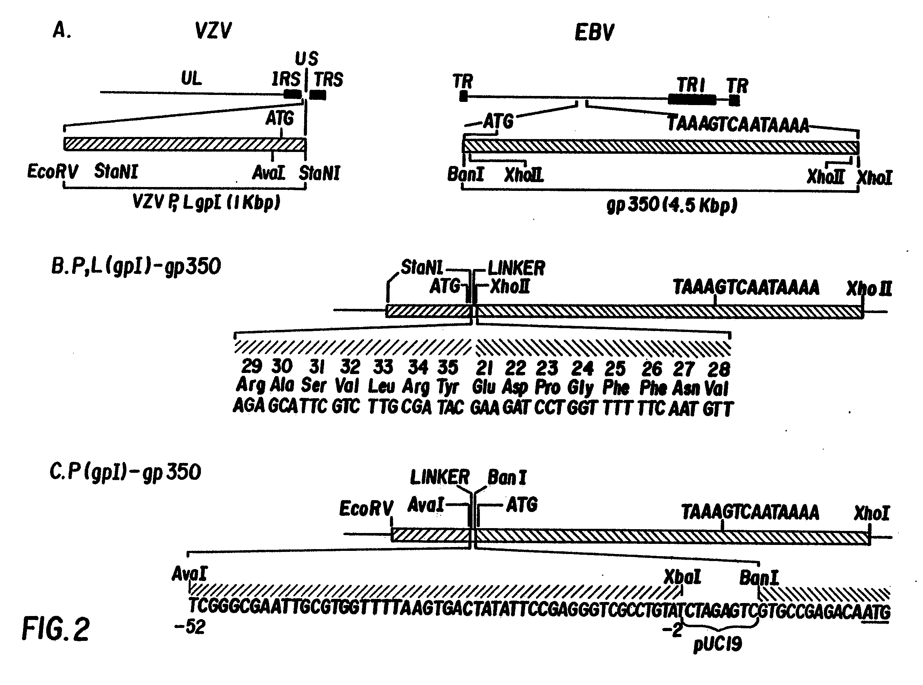

For Vero cell transfectants, two types of varicella-zoster virus (VZV) gpI promoter/EBV gp350 recombinant plasmids were constructed (Fig. 2). VZV P,L(gpI)-gp350 is a VZV promoter-leader/EBV gp350 recombinant which consists of 890 bp of the VZV gpI promoter region, including the first 35 VZV gpI codons fused in frame to EBV gP350 codon 21, [Figure 2B, see also Davison, A.J. EMBO. J. 2, 2203 (1983)]. VZV P(gpI)-gp350 is a recombinant plasmid which consists of 950 bp of the VZV gpI promoter region, extending to 2 bp before the VZV gpI translational initiation codon, fused to the EBV gp350 gene beginning 19 bp upstream of its translational initiation codon (Fig. 2C).

VZV P,L(gpI)-gp350 (see Figure 2B) was constructed by inserting the T4 DNA polymerase-treated, 890bp SfaN1 fragment from pSVGO-12 (a plasmid containing the SacI G fragment of VZV DNA, which includes the VZV gpI gene, [Ellis, R.W. et. al. J. Virol. 53, 81 (1985)]) into the HincII site of pUC19. The EBV BamHI L XhoII fragment containing the gp350/220 gene (Fig. 2A) was then inserted into the BamHI site of the pUC19 polylinker so that the 21st codon of EBV gp350 was separated from the 33rd codon of VZV gpI by two codons of the pUC19 polylinker. These two codons were subsequently replaced by synthetic oligonucleotide linkers to restore VZV gpI codons 34 and 35 (Fig. 2B).

VZV P(gpI)-gp350 (see Figure 2C) was constructed by ligating an 898 bp EcoRV-AvaI VZV gpI fragment to an AvaI-XbaI synthetic oligonucleotide. This restored 50 bp of VZV sequence downstream of the EcoRV-AvaI fragment so that the VZV sequence terminates at -2 bp from the VZV gpI translational initiation site. The resulting EcoRV-XbaI fragment was inserted into the XbaI site of pMA102 (described above and shown in Fig. 1) which places the VZV sequence 14 bp upstream of the complete gp350 gene. The recombination sites in these plasmids were verified by sequencing across the sites using the chain termination method [Sanger, F.A. et. al. J. Mol. Biol. 143, 161 (1980)] with EBV or VZV primers.

For transfection of Mouse Ltk⁻aprt⁻cells, constructions derived in part from cytomegalovirus (CMV) were prepared. pCMVIE-EBMA (see Figure 3A) and pCMVIE-MA-delta (see Figure 3B) were constructed by inserting the 4.4 Kbp BamHI-Bg1II fragment of pMA102 and the 2.6 Kbp BamHI-BcII fragment of pMA-delta-gpt, respectively, into the unique Bg1II site of the expression vector pCMVIE-AK1-DHFR. The vector of Figure 4, pCMVIE-AK1-DHFR, was constructed as follows. Plasmid pBRd [DiMaio, D. et al. Proc. Natl. Acad. Sci. 79, 4030 (1982)] was digested with EcoRV and the ends were modified with an 8-mer synthetic XhoI linker (Collaborative Research). The Tn5 aminoglycoside phosphotransferase (neo) gene under herpes simplex I thymidine kinase (tk) promoter regulation, comprising a ca. 2Kb PvuII segment [Colbere-Garapin, F. et al. J. Mol. Biol. 150, 1 (1982)] was modified by addition at each end of an 8-mer BamHI linker (Collaborative Research) and inserted into the BamHI site of pBRd. At this point, two separate segments were constructed before insertion into pBRd modified with tk-neo sequences, as follows:

(a) the PvuII-EcoRI segment comprising the translation termination and poly (A) signal region of the bovine growth hormone gene (BGH) [Woychik, R. et al. Nucl. Acids Res. 10, 7191 (1982)] was modified at the PvuII end with an 8-mer Bg1II linker (New England Biolabs) and the resulting Bg1II-EcoRI fragment was ligated to

(b) the ClaI-Bg1II segment comprising the major immediate early promoter of cytomegalovirus (CMVIE) [Pasleau, F. et al. Gene 38, 227 (1985)]. The resulting ClaI-EcoRI fragment was blunt-ended and modified at both ends with an XhoI 8-mer linker, and then inserted into the SaII site of pBRd, yielding pBRd modified with tk-neo sequences and now having a unique Bg1II site for insertion of any structural or coding gene between the major immediate early promoter of cytomegalovirus and the translation termination and poly (A) signal region of the bovine growth hormone gene. The final step in the construction of the expression vector PCMVIE-AKI-DHFR involved insertion of a blunt-ended PvuII-BamHI fragment comprising the mouse dihydrofolate reductase (DHFR) gene under SV40 early promoter regulation [Subramani, S. et al. Mol. Cell. Biol. 1, 854 (1981)]. Before insertion, the DHFR gene had been modified from its published form by the sequential deletion of a HindIII site and a Bg1II site (both occurring in non-coding regions), via blunt-ending of sequentially restricted DNA with Klenow DNA polymerase, followed by recircularization; then the modified DHFR gene had its end ligated to the homologous 8-mer synthetic SaII linker. The resulting plasmid is pCMVIE-AK1-DHFR.

## C. Cell culture, transfection, selection and cell sorter analysis

Mouse Ltk ⁻aprt⁻ cells [Wigler, M. et al. Proc. Natl. Acad. Sci. 76, 1373 (1979)] were maintained in Dulbecco's Modified Eagle (DMEM) supplemented with 10% calf serum. Vero cells were maintained in DMEM supplemented with 10% fetal bovine serum (FBS).

For transfection, plasmids were prepared by equilibrium banding in CsCl-ethidium bromide gradients and were at least 90% covalently closed circular DNA. For transfection of Ltk⁻ cells, 1 μg of plasmid DNA (and 19 μg of Ltk⁻ DNA) was applied per plate for 20-24 hr. Cells were exposed to 15% glycerol shock [Lopata, M. et al. Nucl. Acids Res. 12, 5707 (1984)], fed with complete medium and placed in medium with 400μg/ml G418 24 hr. later.

Vero cells were transfected by the calcium phosphate method [Wigler, M. et al., supra] with 10μg of plasmid DNA per T25 flask. DNA was left on the cells for 6 hr. The cells were then fed with non-selective medium and passaged two days later. Vero cells transfected with plasmids containing the gp350 gene downstream of the CMVIE promoter in pSV2-gpt were selected by growth in HAT medium supplemented with 250μg/ml of xanthine and 33μg/ml of mycophenolic acid. Plasmids containing the gp350 gene downstream of the VZV gpI promoter were co-transfected with pSV2-gpt. Ltk⁻ colonies were picked by cylinder-cloning, expanded, and assayed for gp350 expression either by indirect immunofluoresence or by the dot blot assay. Transfected Vero cells were assayed for gp350 expression by indirect immunofluoresence or by the RBC rosetting assay.

The RBC rosetting assay is an in situ red blood cell assay performed by established procedures [Littman, D.R. et. al. Cell 40, 237 (1985)]. The colonies were first incubated with a monoclonal antibody specific for gp350. After washing, they were then incubated with RBCs coated with rabbit anti-mouse IgG. By this sensitive assay, almost all cell colonies were found to be expressing gp350. In a first round of isolation, about 1000 colonies were pooled and cells expressing the most surface gp350 were separated by cell sorting on a FACSII (Becton Dickinson FACS Systems, Mountain View, CA). Briefly, lightly trypsinized cells were stained with specific monoclonal antibody and fluorescein-conjugated goat anti-mouse IgG. Usually about 60% of the cells fluoresced above background. These cells were collected and cultured. As a second round of isolation, the 15% most intensely fluorescent cells were collected and clones were derived by plating at limiting dilution.

One clone of Vero cells obtained by co-transfection with pSV2-gpt and VZV P(gpI)-gp350 was found to express gp350 and gp220. This clone (CL 8.1) expressed more gp350 and gp220 than B95-8 cells (an EBV cell line) induced to replicate EBV, and the electrophoretic mobility was identical to that of gp350 and gp220 obtained from induced B95-8 cells. Expression has been stable for at least a year. Southern blot analysis of the VZV gpI promoter and gp350 sequences in this cell line, using restriction endonucleases which cut upstream of the gpI promoter and within the gp350 gene in the recombinant plasmid that was transfected into these cells, demonstrated that these sequences remain connected in the same size fragment as in the transfected plasmid DNAs.

### EXAMPLE 1

#### PURIFICATION OF SECRETED rEBMA GP350 FROM THE CULTURE MEDIUM OF ALPHA-MATING FACTOR DERIVED RECOMBINANT S. CEREVISIAE, JRY188[pYEBV-1]

The experimental details for the construction of the expression system JRY188[pYEBV-1], as well as its laboratory propagation, are in Schultz, L.D. et al. Gene 54, 113-123 [1987], incorporated by reference for these purposes. The glycoprotein rEBMA gp350 is secreted in significant amounts into the culture medium of JRY188[pYEBV-1].

To grow up large quantities of the expression system, JRY188[pYEBV-1] was cultured in YEHD medium [soypeptone (10 g/L), yeast extract (20 g/L, Difco), dextrose (16 g/L)]. After culture, about 9.5 L of the culture medium was concentrated to 1 L and then diafiltered with 5 L of 0.1 M HEPES, pH 7.4, 0.15 M NaCl (Diafiltration Buffer) using a 0.45 micron membrane. The yeast cells were retained and gp350 permeated through the membrane. The permeate, 14 L, was concentrated to 100 mL and diafiltered with 700 mL of Diafiltration Buffer. The retained solution was removed from the cell and adjusted to 1 mM with respect to $CaCl_2$ and $MnCl_2$ and the entire volume was pumped onto a lentil lectin SEPHAROSE 4B (Pharmacia) affinity column, 5 cm (i.d.) x 24.2 cm, at a flow rate of 80 mL/hr. The column was washed with two bed volumes of Diafiltration Buffer containing 1 mM $CaCl_2$ and 1 mM $MnCl_2$. The column was then eluted with a linear gradient of 0 to 100 mM alpha-methyl-D-mannopyranoside in Diafiltration Buffer, 1 mM $CaCl_2$, 1 mM $MnCl_2$ (total volume of 3 liters). The protein containing fractions were detected by absorbance at 280 nm and combined into a pool of 515 mL. The pool was concentrated to 30.5 mL and diafiltered with 200 mL of 20 mM Tris, pH 8.0. The concentrated, diafiltered pool was pumped onto a MONO-Q anion exchange column (Pharmacia) operated on a Pharmacia FPLC system. The column was eluted with 20 mM Tris, ph 8.0, until no additional UV absorbing material was released and then the gp350 was eluted with a linear gradient of 0 to 300 mM NaCl in 20 mM Tris, pH 8.0. Fractions, 8.0 mL each, were assayed for the presence of gp350 by immunoblotting and a pool was made of those fractions containing gp350. The pool was concentrated to 18 mL and a protein concentration of 1.32 mg/mL. This preparation of purified rEBMA gp350 contained one component detectable by immunoblotting and was approximately 90% pure as judged by silver staining.

### EXAMPLE 2

#### PURIFICATION OF RECOMBINANT EPSTEIN-BARR MEMBRANE ANTIGEN GP350 OR GP220 FROM VERO CELLS

Clone CL8.1 was grown in selective media and stored as wet pack at -70° C in 1-2 gm aliquots. A sample of 6.65 gm of cells were suspended in 3 ml Extraction Buffer (2% TRITON X-100, 0.15M NaCl in 20mM Tris-HCl, pH 7.4) for every gm of cells. Phenylmethylsulfonylfluoride (PMSF) was added to a concentration of 2mM. The cell suspension was placed into 50 ml tubes, packed in ice and sonication was performed, followed by clarification at 8500 rpm for 40 minutes. The supernatant contained 172 mg of protein, yielding a cell extract.

A 2.5 cm x 2.0 cm column of lentil lectin SEPHAROSE-4B was prepared by washing with 100 ml of Eluting Buffer (0.1% TRITON X-100, 1mM MnCl₂, 1mm CaCl₂, 0.15M NaCl, 0.2M α-methylmannoside in 20mM Tris-HCl, pH 7.4) at a flow rate of 10 ml/hr. A volume of 50ml Column Buffer (0.1% TRITON X-100, 1mM MnCl₂, 1mM CaCl₂, 0.15M NaCl in 20mM Tris-HCl, pH 7.4) was then washed through. The cell extract was loaded onto the column and the unadsorbed proteins washed with 10 bed volumes of Column Buffer. The adsorbed antigen material was desorbed with Eluting Buffer and collected in 1 ml fractions. The eluted fractions found to contain antigen by SDS-PAGE, silver stain and protein dot blots were pooled with a recovery of 5.58 mg in 14.5 ml, yielding an eluted antigen pool.

The eluted antigen pool was then concentrated and diafiltrated as follows: The eluted antigen pool was mixed with an equal volume of Diafiltration Buffer (0.8% CHAPS, 20mM DTT in 20mM Tris-HCl, pH 8.0) in order to create smaller "mixed" detergent micelles. The purpose of making "mixed" micelles was to facilitate the exchange of CHAPS detergent for TRITON X-100 by diafiltration. The antigen pool was then concentrated to 30 ml and diafiltered with 300 ml Diafiltration Buffer to remove small molecular weight proteins and TRITON X-100. The diafiltered concentrate contained 1.98 mg of protein in 6.6 ml (35% of the material before diafiltration).

A MONO-Q HR10/10 column (Pharmacia) was prepared on a FAST PROTEIN LIQUID CHROMATOGRAPHY (Pharmacia FPLC) system by washing with Buffer A (0.8% CHAPS in 20mM Tris-HCl, pH 8.0). The diafiltered concentrate was loaded in 2.2 ml aliquots and the unadsorbed proteins washed off with Buffer A. The adsorbed proteins were eluted in 1 ml fractions with a 0-0.5M NaCl gradient. The gp350 material eluted at 0.23M to 0.26M, and was pooled and concentrated 33-fold. Protein recovery was 7% of the load to the MONO-Q column.

The resulting concentrated antigen pool was subjected to gel filtration as follows: A SUPEROSE 6 HR10/30 column was prepared by washing with Buffer A. 200 microliter aliquots of the concentrated antigen pool was loaded per run and 1 ml fractions collected. Fractions which were determined to contain recombinant gp350 by immunoblotting were pooled and concentrated. Silver stained gels and immunoblots showed the material to have 90% or greater recombinant gp350 or gp220.

EXAMPLE 3

PURIFICATION OF rEBMA GP350 OR GP220 FROM EXPRESSOR MOUSE LTK⁻ CELLS

A 3.6 gm batch of frozen mouse L cells transfected with pCMVIE-EBMA, positive by immunofluoresence for EBMA, were thawed and resuspended in 10.8 mL of Extraction Buffer containing 2 mM PMSF. The cell suspension was immersed in ice in a 50 ml polycarbonate centrifuge tube, and then sonicated in five 20 second pulses. The mixture was then clarified by centrifuging 40 minutes, 8,500 xg, 4°C in a refrigerated centrifuge. The clarified supernatant medium was decanted, the cell pellet resuspended in the original volume of Extraction Buffer and the sonication and clarification repeated. Decanted supernatants were then combined.

Recombinant EBMA was then isolated from the combined decanted supernatant by affinity chromatography on lentil lectin SEPHAROSE 4B and con A SEPHAROSE 4B followed by anion exchange chromatography on MONO-Q (Pharmacia) and gel-filtration chromatography on SUPEROSE 12 (Pharmacia), as follows:

A column of lentil lectin SEPHAROSE 4B (Pharmacia) having 10 mL bed volume was packed and prepared for use by eluting with 100 mL of Eluting Buffer (0.1% TRITON X-100, 1 mM MnCl₂, 1 mM CaCl₂, 0.15 M NaCl, 200 mM alpha-D-methylmannoside) followed by 50 mL of Column Buffer (0.1% TRITON X-100, 1mM MnCl₂, 1 mM CaCl₂, 0.15 NaCl). A 20 mL sample of the decanted supernatant liquid was charged to the column and the unadsorbed proteins were eluted with Column Buffer until no additional protein could be removed. The column was then eluted with Eluting Buffer. However, in this case the rEBMA was in the unadsorbed protein fraction and it was further purified by con A affinity chromatography, as follows:

A column of con A SEPHAROSE 4B (Pharmacia), 10 mL bed volume, was packed and washed with Eluting Buffer followed by Column Buffer. A 20 mL sample of the unadsorbed protein fraction from the lentil lectin column was charged to the con A column and it was washed with Column Buffer until no additional protein was eluted. The adsorbed protein column was eluted with Eluting Buffer. The column effluents were collected in 2 mL fractions and each was monitored for the presence of rEBMA by protein dot blotting and silver staining. The rEBMA positive fractions were combined into a 12.5 mL pool. Fractions containing rEBMA were those eluted by Eluting Buffer and these were combined into a 12.5 mL pool, resulting in con A purified rEBMA.

A five mL aliquot of con A purified rEBMA was further purified by QAE-anion exchange chromatography on a MONO-Q column (Pharmacia) operated on a FAST PROTEIN LIQUID CHROMATOGRAPHY (FPLC) system. The MONO-Q column (5 mm x 5 cm) was equilibrated with Starting Buffer (0.1% TRITON X-100, 20 mM Tris, pH8). The five mL aliquot of con A purified rEBMA was Prepared for MONO-Q QAE anion-exchange chromatography by exchanging the buffer on a pre-packed column of SEPHADEX G-25 (PD-10; Pharmacia) that had been equilibrated with Starting Buffer. The unadsorbed proteins from an applied sample were eluted with Starting Buffer after which the adsorbed proteins were eluted with a linear gradient of 0-0.5 M NaCl in Starting Buffer (total volume of the gradient was 20 mL). SDS-PAGE analysis showed that rEBMA was eluted with 0.3-0.38 M NaCl and these fractions were combined to make a 3 mL pool, resulting in MONO-Q purified rEBMA.

MONO-Q purified rEBMA was subjected to a final fractionation step of gel filtration, as follows: An analytical column of SUPEROSE 6 (30 mL bed volume) using the FPLC was equilibrated with 0.15 M NaCl, 0.1% TRITON X-100, 20 mM Tris-HCl, pH 8 by passing 150 mL of buffer through it at 1.0 mL/min. One-half mL aliquots of the MONO-Q purified rEBMA were fractionated by gel filtration and 1.0 mL fractions of effluent were collected. Fractions were monitored by silver staining and immunoblotting after SDS-PAGE and a pool was made of those fractions which were positive for rEBMA. This final fractionation step resulted in a yield of 0.1 mg of rEBMA per gram of cells (wet weight) with greater than 90% purity.

EXAMPLE 4

PURIFICATION OF RECOMBINANT EPSTEIN-BARR ANTIGEN GP350 OR GP220 FROM B95-8 MARMOSET LYMPHOBLASTOID CELL LINE.

Marmoset lymphoid cells (B95-8) producing Epstein-Barr virus (EBV) and positive by immunofluorescence for membrane antigens gp350 or gp220 (EBMA) were cultured in medium containing penicillin (100 I.U./mL), streptomycin (100 mcg/mL) and 5% fetal calf serum (FCS).

These cells were induced by incubation with TPA (12-0-Tetradecanoyl-phorbol-13-acetate, Sigma, Poole, U.K.) as follows: TPA was stored at a concentration of 2 mg/mL in dimethyl sulphoxide in ampoules filled with nitrogen at -20°C, and was diluted for use in warm sterile phosphate-buffered saline (PBS) immediately prior to addition to the culture. Cultures for induction were prepared by seeding flasks with $5 \times 10^5$ cells/mL, then incubating overnight. TPA and Na butyrate (Sigma B5887) was added to cultures with final concentrations of 10mM and 200 ng/mL, respectively, followed by another incubation for 72 hrs. Cells were harvested for storage by spinning at 2000 rpm 10 min, washing 2 x in PBS and freezing the pellet.

Frozen B95-8 cell pellets, 6.5 g wet weight, were thawed and suspended in Extraction Buffer (2% TRITON X-100, 0.15 M NaCl, 20 mM Tris, pH 7.4) at a ratio of one gram of cells to three milliliters of buffer. A stock solution of 200 mM phenylmethylsulfonyl fluoride was added to a final concentration of 2 mM to inhibit serine proteases and the cell suspension was aliquoted in 35 mL portions into 50 mL polycarbonate centrifuge tubes. The tubes were immersed in ice and the mixture was sonicated in five 20 second pulses. The mixture was then clarified by centrifuging 40 minutes, 8,500 xg, 4°C, in a refrigerated centrifuge. The clarified supernatant medium was decanted, the cell pellet resuspended in the original volume of Extraction Buffer and the sonication and clarification repeated. The decanted supernatant medium was combined into a single pool and fractionated by lentil lectin chromatography.

A column of lentil lectin SEPHAROSE 4B (Pharmacia) having 10 mL bed volume was packed and prepared for use by eluting with 100 mL of Eluting Buffer (0.1% TRITON X-100, 1 mM MnCl₂, 1 mM CaCl₂, 0.15 M NaCl, 200 mM alpha-D-methylmannoside in 20 mM Tris, pH 7.4) followed by 50 mL of Column Buffer (0.1% TRITON X-100, 1 mM MnCl₂, 1 mM CaCl₂, 0.15 NaCl in 20 mM Tris pH 7.4.) Then a portion of the pool of decanted supernatant medium (13 mL containing 195 mg of protein) was applied to the column. When all the solution had entered the column the unadsorbed proteins were washed away with 50 mL of Column Buffer and the adsorbed fraction desorbed with 50 mL of Eluting Buffer. The effluent from the column was collected in one mL fractions that were monitored for EBMA by immunoblotting. EBMA was present in selected eluted fractions and a pool of these fractions was made. It contained 2.52 mg of protein in 8 mL.

EBMA obtained by fractionation with lentil lectin affinity chromatography was further purified by QAE-anion exchange chromatography on a MONO-Q column (Pharmacia) operated on a FAST PROTEIN LIQUID CHROMATOGRAPHY (FPLC) system. The MONO-Q column 5 mm x 5 cm) was equilibrated with Starting Buffer (0.1% TRITON X-100, 20 mM Tris, pH 8). A sample of the EBMA containing pool (5.0 mL) obtained by lentil lectin affinity chromatography was prepared for MONO-Q anion-exchange chromatography by exchanging the buffer on a pre-packed column of SEPHADEX G-25 (PD-10; Pharmacia) that had been equilibrated with Starting Buffer. The unadsorbed proteins of an applied sample were eluted with Starting Buffer after which the adsorbed proteins were eluted with a linear gradient of 0-0.5 M NaCl in Starting Buffer (total volume of the gradient was 20 mL). Immunoblotting and silver staining assays showed that EBMA was in the fractions eluting at 0.25-0.30 M NaCl. A pool of purified EBMA was collected and further fractionated by gel-filtration chromatography on an analytical column of SUPEROSE 6 (30 ml bed volume) using the FPLC. The column was equilibrated with 0.15 M NaCl, 0.1% TRITON X-100, 20 mM Tris-HCl, pH 8 by passing 150 mL of buffer through it at 1.0 mL/min. One-half mL aliquots of the EBMA containing pool obtained by MONO-Q chromatography were fractionated by gel-filtration and 1.0 mL fractions of effluent were collected. Fractions were monitored by silver staining and protein dot blotting after SDS-PAGE and a pool was made of those fractions which were positive for EBMA.

This procedure yielded 11 mcg of EBMA/gram of B95-8 cells (wet weight), with a purity > 90% pure as assessed by SDS-PAGE (See Materials and Methods, supra).

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims.

**Claims**

1. A process for purifying recombinant EBV membrane antigens from a recombinant mammalian expression system, comprising the steps of:

(a) disrupting VERO cells expressing recombinant EBV membrane antigens;

(b) subjecting the disruptate to one or two steps of lectin affinity chromatography, yielding eluted fractions containing EBV membrane antigen;

(c) subjecting said eluted fractions to gel filtration chromatography, yielding purified recombinant EBV membrane antigens.

2. The process of claim 1 wherein said Vero cells expressing recombinant EBV membrane antigens are CL 8.1 cells.

3. The process of claim 1 wherein said step (b) comprises treating the disruptate to one step of lectin affinity chromatography with a lentil lectin column.

4. A process for purifying recombinant gp350 of EBV from recombinant Vero cells, comprising the steps of

(a) disrupting CL 8.1 cells expressing recombinant gp350 or gp220;

(b) clarifying the disruptate;

(c) subjecting the clarified supernatant to one step of lentil lectin chromatography, and collecting an eluted gp350 or gp220 pool;

(d) subjecting said eluted gp350 or gp220 to one step of anion exchange column chromatography;

(e) subjecting fractions containing gp350 or gp220 to gel filtration, yielding purified recombinant gp350 or gp220.

5. A process for purifying recombinant EBV membrane antigens secreted from a recombinant yeast expression system, comprising the steps of:

(a) collecting and dialyzing a culture medium contacted with yeast cells secreting rEBMA, yielding a retentate containing rEBMA;

(b) subjecting said retentate to one or two steps of lectin affinity chromatography, yielding eluted fractions containing rEBMA;

(c) subjecting said eluted fractions to anion exchange chromatography, yielding purified yeast-derived rEBMA.

6. A process for purifying non-secretory recombinant EBV membrane antigens from a recombinant yeast expression system, comprising the steps of:

(a) collecting yeast cells expressing rEBMA;

(b) disrupting said cells;

(c) removing debris, yielding a clarified supernatant;

(d) dialyzing the clarified supernatant, yielding a retentate;

(e) subjecting the retentate to one or more steps of lectin affinity chromatography, yielding eluted fractions containing rEBMA;

(f) subjecting said eluted fractions to anion exchange chromatography, yielding purified yeast-derived rEBMA.

7. The process of claim 5 wherein said yeast cells secreting rEBMA are JRY188[pYEBV-1].

8. The process of claim 5 wherein step (b) comprises subjecting the retentate to one step of lectin affinity chromatography.

9. The process of claim 5 wherein the apparatus for the anion exchange chromatography of step (c) comprises a MONO-Q anion exchange column.

10. A process for purifying recombinant EBV gp350 or gp220 secreted from a recombinant yeast expression system, comprising the steps of:

(a) collecting and dialyzing a culture medium contacted with JRY188[pYEBV-1], yielding fractions containing recombinant EBV gp350 or recombinant EBV gp220 ;

(b) subjecting the product of step (a) to lectin affinity chromatography, and eluting the bound fraction;

(c) passing the bound fraction through an anion exchange column, yielding purified recombinant EBV gp350 or gp220 derived from yeast.

11. A process for purifying recombinant EBV membrane antigens from a recombinant mammalian expression system comprising the steps of:

(a) disrupting mouse L cells expressing recombinant EBV membrane antigens;

(b) subjecting the disruptate to one or two steps of lectin affinity chromatography, yielding eluted fractions containing EBV membrane antigen;

(c) subjecting said eluted fractions to anion exchange chromatography, yielding chromatographed fractions containing recombinant EBV membrane antigens; and

(d) subjecting said chromatographed fractions to gel filtration chromatography, yielding purified recombinant EBV membrane antigens.

12. The process of claim 11 wherein said mouse L cells expressing recombinant EBV membrane antigens are mouse L cells transfected with pCMVIE-EBMA and expressing rEBMA.

13. The process of claim 11 wherein said step (b) comprises subjecting the cell disruptate to clarification, then adsorbing out undesired contaminants by passing through a lentil lectin column, and treating the unadsorbed protein fractions to con A gel chromatography, and collecting eluted protein fractions that adsorbed to Con A and contain rEBMA.

A. pMAIO2

XhoI  HindⅢ        gp350        ScaI  SmaI        BglⅡ  HindⅢ        pUC19

                    pUC19 ←──→ EBV                    XhoI
                                                              Ap

<u>GGATCC</u> <u>TCTAGA</u>GTC<u>GTGCCGAGACA</u><u>ATG</u>
<u>BamHI</u>  <u>XbaI</u>

B. pMA△-gpt

BamHI        gp350                    (BglⅡ/BamHI)        pSV2-gpt

                                                              Ap        SV2-gpt

        <u>Val</u>  *    *    *
        AGTTTAAATAGC<u>TGATCA</u>GCTATTTAAAACT
EBV ←──→                    <u>BclI</u>                    ←──→

FIG. 1

EP 0 312 164 A1

FIG. 2

EP 0 312 164 A1

A. pCMVIE-EBMA

CMVIE    gp350                      BGH poly A—TK-NEO→        —SV2-DHFR→        pBRd

(BglII/BamHI)                    BglII   BamHI        BamHI           BamHI      Ap

B. pCMVIE-MAΔ

CMVIE        MAΔ    BGH poly A—TK-NEO→        —SV2 DHFR→        pBRd

(BglII/BamHI)  (BclI/BglII)  BamHI       BamHI            BamHI      Ap

FIG. 3

EP 0 312 164 A1

FIG. 4

```
YEAST CULTURE                    YEAST CULTURE
SECRETING rEBMA                  EXPRESSING rEBMA
                                 WITHOUT SECRETION
       │                                │
       ▼                                ▼
COLLECTION OF CELLS             COLLECTION OF CELLS
       │                                │
   ┌───┴───┐                    ┌───────┴───────┐
   ▼       ▼                    ▼               ▼
MEDIUM    CELLS                SUP            CELLS
   │                                            │
   │                                            ▼
   │                           DISRUPTION OF CELLS
   │                           REMOVAL OF DEBRIS
   │                                   │
   │                              ┌────┴────┐
   │                              ▼         ▼
   │                             SUP      DEBRIS
   │                              │
   └──────────┬───────────────────┘
              ▼
          DIALYSIS              MAMMALIAN CELL
              │                    CULTURE
              ▼                       ┊
          LECTIN  ◄┄┄┄┄┄┄┄┄┄┄┄   DISRUPTION OF
         AFFINITY               CELLS, REMOVAL
              │                   OF DEBRIS
PURIFIED      ▼
YEAST─  ◄   ANION
DERIVED    EXCHANGE
rEBMA         ┊
              ▼
            GEL
         FILTRATION
              ┊
              ▼
     PURIFIED MAMMALIAN
     CELL-DERIVED rEBMA
```

FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF VIROLOGY, vol. 61, no. 5, May 1987, pages 1416-1420; C.R. NEMEROW et al.: "Identification of gp350 as the viral glycoprotein mediating attachment of Epstein Barr Virus (EBV) to the EBV/C3d receptor of B cells: Sequence homology of gp350 and C3 complement fragment C3d" * Page 1416, column 2, lines 14-30 * | 1-3,5-8,10-12 | C 12 P 21/00 C 12 N 15/00 A 61 K 39/245 |
| Y | IDEM. | 4,9,13 | |
| X | EP-A-0 001 365 (MERCK & CO. INC.) * Page 1, line 1 - page 2, line 4; pages 7,8; page 10, lines 1-4 * | 1-8,10-13 | |
| Y | CHEMICAL ABSTRACTS, vol. 94, no. 17, 27th April 1981, page 583, abstract no. 137591d, Columbus, Ohio, US; B.C. STRNAD et al.: "Biochemical characterization of Epstein-Barr virus membrane antigen-associated glycoproteins", & BIOCHEM. BIOPHYS. RES. COMMUN. 1981, 98(4), 1121-7 * Abstract * | 4,15 | |
| Y | THE JOURNAL OF IMMUNOLOGY, vol. 129, no. 2, August 1982, pages 814-818, US; L.F. QUALTIERE et al.: "Purification and biologic characterization of a major Epstein Barr virus-induced membrane glycoprotein" * Page 814, column 2, lines 44-60; page 815, column 1, lines 1-12 * -/- | 1-13 | TECHNICAL FIELDS SEARCHED (Int. Cl.4) C 12 N C 12 P A 61 K |

The present search report has been drawn up for all claims

| Place of search | Date of completion of the search | Examiner |
|---|---|---|
| THE HAGUE | 25-01-1989 | SKELLY J.M. |

EPO FORM 1503 03.82 (P0401)

European Patent Office

**EUROPEAN SEARCH REPORT**

Application Number

EP 88 20 2242

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GENE, vol. 54, no. 1, 1987, pages 113-123, Amsterdam, NL; L.D. SCHULTZ et al.: "Expression and secretion in yeast of a 400-kDa envelope glycoprotein derived from Epstein-Barr virus"<br>--- | | |
| A | VACCINES 87, MODERN APPROACHES TO NEW VACCINES: PREVENTION OF AIDS AND OTHER VIRAL, BACTERIAL AND PARASITIC DISEASES, CONF. COLD SPRING Harbour, New York, 1986, pages 412-418; E.A. EMINI et al.: "Antigenic analysis of the Epstein-Barr virus major membrane protein (gp350/gp220) expressed in yeast and mammalian cells"<br>--- | | |
| P,X | VIROLOGY, vol. 166, 1988, pages 387-393, Academic Press Inc.; E.A. EMINI et al.: "Antigenic analysis of the Epstein-Barr virus major membrane antigen (gp350/220) expressed in yeast and mammalian cells: Implications for the development of a subunit vaccine"<br>* Whole document, especially page 387, column 1, lines 31-38 *<br>--- | 1-13 | |
| P,Y | JOURNAL OF IMMUNOLOGICAL METHODS, vol. 108, 1988, pages 231-236, Elsevier Science Publishers B.V.; E.M. DAVID et al.: "Efficient purification of Epstein-Barr virus membrane antigen gp340 by fast protein liquid chromatography"<br>* Whole document *<br>---      -/- | 9 | TECHNICAL FIELDS SEARCHED (Int. Cl.4) |

The present search report has been drawn up for all claims

| Place of search | Date of completion of the search | Examiner |
|---|---|---|
| THE HAGUE | 25-01-1989 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | J. GEN. VIROL., vol. 64, 1983, pages 455-460, SGM, GB; A.J. MORGAN et al.: "Purification and properties of the gp340 component of Epstein-Barr virus membrane antigen in an immunogenic form" * Page 456, legend figure 2 * | 1-13 | |

TECHNICAL FIELDS
SEARCHED (Int. Cl. 4)

The present search report has been drawn up for all claims

| Place of search | Date of completion of the search | Examiner |
|---|---|---|
| THE HAGUE | 25-01-1989 | SKELLY J.M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
& : member of the same patent family, corresponding
   document

EPO FORM 1503 03.82 (P0401)